(19) Europäisches Patentamt
European Patent Office
Office européen des brevets

(11) Veröffentlichungsnummer: **0 603 755 A2**

# (12) EUROPÄISCHE PATENTANMELDUNG

(21) Anmeldenummer: **93120375.6**

(22) Anmeldetag: **17.12.93**

(51) Int. Cl.5: **C07D 501/20**, A61K 31/545, C07D 501/46, //C07D333/38

(30) Priorität: **24.12.92 DE 4244069**

(43) Veröffentlichungstag der Anmeldung: **29.06.94 Patentblatt 94/26**

(84) Benannte Vertragsstaaten: **AT BE CH DE DK ES FR GB GR IE IT LI LU MC NL PT SE**

(71) Anmelder: **HOECHST AKTIENGESELLSCHAFT**
**Brüningstrasse 50**
**D-65929 Frankfurt am Main(DE)**

(72) Erfinder: **Dr. Dürckheimer, Walter**
**Im Lerchenfeld 45**
**D-65795 Hattersheim(DE)**
Erfinder: **Dr.Isert, Dieter**
**In den Weingärten 1**
**D-65760 Eschborn(DE)**
Erfinder: **Dr. Rippel Robert**
**Frankfurter Strasse 66**
**D-65719 Hofheim/Taunus(DE)**
Erfinder: **Dr.Rangoonwala, Ramazan**
**Brandenburger Weg 18**
**D-65719 Hofheim/Taunus(DE)**
Erfinder: **Dr. Dornauer, Horst**
**Oranienstrasse 78**
**D-65812 Bad Soden(DE)**

## (54) Cephalosporinsalze und Verfahren zu deren Herstellung.

(57) Gegenstand der Erfindung sind Cephalosporinsalze der allgemeinen Formel (I),

$[\text{Cephalosporin}]^{m\ominus}\ x[BxH^+]^{\oplus}_n$ (I)

worin das salzbildende Anion ein antibiotisch wirksames Cephalosporin mit einer zur Anionbildung befähigten Gruppe (m = 1) oder auch mehreren Gruppen dieser Art (m = 2 oder 3) enthält und die Base, die als reines (+) oder (-) Enantiomer oder in racemischer Form vorliegen kann, die allgemeine Formel (B) besitzt,

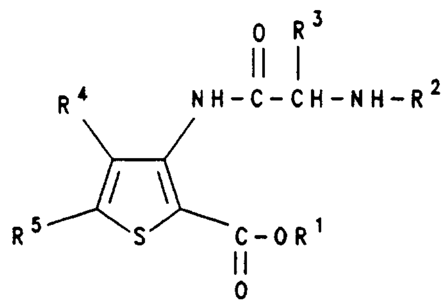

(B)

worin

EP 0 603 755 A2

| | |
|---|---|
| $R^1$ | gerade oder verzweigtes Alkyl mit 1-8 C-Atomen, welches gegebenenfalls substituiert ist durch Hydroxy, Phenyl oder durch Phenyl, das durch Alkoxy oder Halogen substituiert ist; vorzugsweise eine Methyl-, Ethyl-, Propyl-, Butyl- oder Benzyl-Gruppe bedeutet, |
| $R^2$, $R^3$, $R^4$ und $R^5$ | gleich oder verschieden sein können und Wasserstoff, gerade oder verzweigtes Alkyl mit 1-4 C-Atomen bedeuten, vorzugsweise ist $R^2$ gleich Propyl, $R^3$, $R^4$ gleich Methyl und $R^5$ gleich Wasserstoff und |
| m und n | je nach Zahl der salzbildenden Gruppen im Cephalosporinteil eine Zahl von 1 bis 3 für m, vorzugsweise 1 und 2, und |
| für n | 0,1 bis 3, vorzugsweise 0,1 bis 2 bedeuten. |

Die erfindungsgemäßen Cephalosporinsalze besitzen eine gute antibakterielle Wirksamkeit und können daher als Arzneimittel verwendet werden.

Die Erfindung betrifft neue Salze von Cephalosporinen, die eine gute antibakterielle Wirksamkeit besitzen und sich aufgrund ihrer vorteilhaften pharmakokinetischen und physikochemischen Eigenschaften zur parenteralen, besonders zur intramuskulären Anwendung in der Human- und Veterinärmedizin eignen.

Gegenstand der Erfindung sind Cephalosporinsalze der allgemeinen Formel (I),

$$[\text{Cephalosporin}]^{m\ominus}\ x[BxH^+]^{\oplus}_n \qquad (I)$$

worin das salzbildende Anion ein antibiotisch wirksames Cephalosporin mit einer zur Anionbildung befähigten Gruppe (m = 1) oder auch mehreren Gruppen dieser Art (m = 2 oder 3) enthält und die Base, die als reines (+) oder (-) Enantiomer oder in racemischer Form vorliegen kann, die allgemeine Formel (B) besitzt,

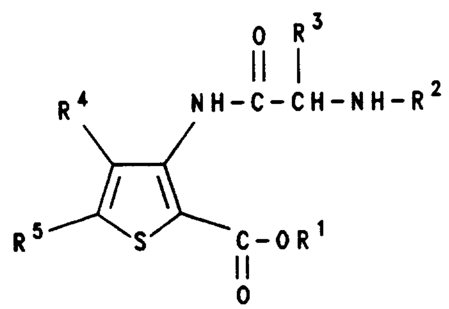

(B)

worin

| | |
|---|---|
| $R^1$ | gerade oder verzweigtes Alkyl mit 1-8 C-Atomen, welches gegebenenfalls substituiert ist durch Hydroxy, Phenyl oder durch Phenyl, das durch Alkoxy oder Halogen substituiert ist; vorzugsweise eine Methyl-, Ethyl-, Propyl-, Butyl- oder Benzyl-Gruppe bedeutet, |
| $R^2$, $R^3$, $R^4$ und $R^5$ | gleich oder verschieden sein können und Wasserstoff, gerade oder verzweigtes Alkyl mit 1-4 C-Atomen bedeuten, vorzugsweise ist $R^2$ gleich Propyl, $R^3$, $R^4$ gleich Methyl und $R^5$ gleich Wasserstoff und |
| m und n | je nach Zahl der salzbildenden Gruppen im Cephalosporinteil eine Zahl von 1 bis 3 für m, vorzugsweise 1 und 2, und |
| für n | 0,1 bis 3, vorzugsweise 0,1 bis 2 bedeuten. |

Als Cephalosporin-Komponenten eignen sich alle Cephalosporine, die aufgrund ihres antibakteriellen Spektrums, ihrer pharmakokinetischen Eigenschaften und Verträglichkeit bei Mensch und Tier als Antibiotika eingesetzt werden können und zur Salzbildung befähigt sind. Sie können in 7-Stellung ein Wasserstoffatom oder eine 7$\alpha$-Methoxygruppe (Cephamycin-Typ) tragen. Ebenso kann das Schwefelatom des Cephalosporingerüstes durch ein Sauerstoffatom (1-Oxacephalosporine) oder eine Methylengruppe (1-Carbacephalosporine) ersetzt sein. Beispiele für Cephalosporine, welche die erfindungsgemäßen Salze bilden können sind die folgenden, bereits beschriebenen Verbindungen:

Cefamandol, Cefazolin, Cefoperazon, Cefbuperazon, Cefdaloxim, Cefdenir, Cefodizim, Cefixim, Cefmenoxim, Cefminox, Cefonicid, Cefotaxim, Ceforamid, Cefotetan, Cefotiam, Cefoxitin, Cefpimizol, Cefpiramid, Ceftazidim, Cefteram, Ceftezol, Ceftibuten, Ceftiofur, Ceftizoxim, Ceftriaxon, Cefuroxim, Cefuzonam, Cephalothin, FCE 20485, Me 1206, E-0702, CP 0467, GR 69153, SR 44337, Ro 09-1428, Ro 244383, Ro 249424, Latamoxef, Flomoxef, CS 807, S 1090, Fk 037.

Bevorzugt sind 2-Aminothiazol-Cephalosporin-Antibiotika der 3. Generation. Besonders bevorzugte Cephalosporin-Komponenten sind: Cefodizim, Cefotaxim, Ceftizoxim, Ceftriaxon, Cefazolin und Cefuroxim.

Die Basenkomponente B der erfindungsgemäßen Salze ist in einer bevorzugten Ausführungsform das Anästhetikum (±) Articain (B; $R^1 = R^3 = R^4 = CH_3$; $R^2 = CH_2CH_2CH_3$; $R^5 = H$), das in der Literatur auch als Ultracain oder als Carticain bezeichnet wird [vgl. Merck Index, 11. edition (1989), S. 285, #1878]. Als besonders geeignet zur Salzbildung haben sich die beiden Enantiomeren von Articain, nämlich das (+) Articain und das (-) Articain erwiesen, die als freie Basen und als Salze in reiner Form erhältlich und einsetzbar sind. Beispiele für den Einsatz und die Herstellung dieser Enantiomeren gibt der experimentelle

Teil.

Die Strukturanaloga des Articains, mit den in der Formel (B) angegebenen Substituenten sind in den Eigenschaften mit Articain eng verwandt und besitzen wie dieses die Fähigkeit der Salzbildung mit Cephalosporinen. Als Articain-Analoga sind im Prinzip die in der deutschen Patentschrift DE 1 643 325 genannten Thiophenderivate einsetzbar.

Die Herstellung der erfindungsgemäßen Salze erfolgt nach an sich bekannten Methoden, z. B. indem man ein Cephalosporin (C) als freie Säure mit der Basenkomponente (B) im gewünschten Verhältnis mischt, vorzugsweise in gelöster oder suspendierter Form in Wasser, organischen Lösungsmittel, wie z. B. Methanol oder Lösungsmittelgemischen. Die Menge und das Mischungsverhältnis der Lösungsmittel richtet sich nach der Löslichkeit der Ausgangskomponenten. Beim Mischen ist darauf zu achten, daß die Neutralisations- und Mischungswärme in geeigneter Weise, z. B. durch Kühlen und Rühren, abgeführt wird. Die Isolierung erfolgt in üblicher Weise bei schwerlöslichen Salzen durch Abfiltrieren, bei leichter löslichen Salzen durch Einengen der Lösung oder Gefriertrocknung.

Eine andere Herstellungsvariante ist die doppelte Umsetzung der Cephalosporinkomponente und des Articainderivates (B) in Form in Wasser leichtlöslicher Ausgangskomponenten, gegebenenfalls unter Zusatz eines mit Wasser mischbaren inerten organischen Lösungsmittels. Die Cephalosporine können auch als freie Säuren eingesetzt werden, und daraus durch Zugabe einer Base, wie z.B. $NaHCO_3$, $NaCO_3$, NaOH, KOH, $NH_4OH$ direkt die Lösung des Cephalosporins gewonnen werden. Gegenstand der Erfindung sind auch Salze eines Cephalosporins, die mehrere verschiedene Kationen-Komponenten enthalten, beispielsweise Articain-Kationen vom Typ B und zusätzlich anorganische Kationen wie beispielsweise Alkali- oder Erdalkalimetall-Ionen. Die in der allgemeinen Formel (I) enthaltenen Zahlen m und n müssen daher nicht gleich sein, da zum Ausgleich der negativen Ladung der Cephalosporin-Anionen auch Kationen vorhanden sein können, die nicht von einer anästhetisch wirksamen Basenkomponente (B) abgeleitet sind. Dies gilt insbesondere für Cephalosporine, wie z.B. Ceftriaxon oder Cefodizim, die zwei acide Gruppen zur Salzbildung enthalten.

Die Herstellungsbedingungen bezüglich Lösungsmittel, Konzentration und Temperatur können wegen der günstigen physikochemischen Eigenschaften der Salze weitgehend variiert werden. Die Kristallgröße läßt sich durch entsprechende Wahl der Reaktionsbedingungen steuern. Langsames Auskristallisieren bei Raumtemperatur liefert größere Kristalle, während Kühlen und rasches Zusammengeben der Komponenten zu kleineren Kristallen führt. Gegebenenfalls können die erfindungsgemäßen Salze durch Umkristallisieren oder Umfällen gereinigt werden, wobei sich die Wahl und Menge der Lösungsmittel von der Natur der eingesetzten Komponenten und vom Reaktionsprodukt abhängt. Eine einheitliche Korngröße der beanspruchten Salze läßt sich auch durch Mahlen erreichen.

Überraschenderweise wurde gefunden, daß bei Umsetzung von Cephalosporinen mit racemischem (±) Articain bzw. seinen Derivaten, vorwiegend das schwerlösliche (+) Articainsalz des Cephalosporins als reines diastereomeres Salz ausfällt und das leichter lösliche Diastereomere des (-) Articains zunächst in Lösung bleibt und erst beim Kühlen oder Einengen ausfällt. Dieses kann auch durch Gefriertrocknung isoliert werden.

Die Cefuroxim- und Cefazolin-Salze von (+) Articain und (-) Articain besitzen jedoch eine ähnliche Löslichkeit. In diesem Falle ist es daher vorteilhaft, die enantiomerreinen Basenkomponenten B einzusetzen, wenn ein einheitliches diastereomeres Salz gewünscht wird.

Mit steigender Lipophilie der Base B, z. B. bei Verlängerung des Alkylrestes $R^1$ nimmt die Wasserlöslichkeit der Salze ab und die Löslichkeit in organischen Lösungsmittel, wie z. B. Methanol, Äthanol, DMF oder DMSO zu.

Die erfindungsgemäßen Salze sind in der Regel in fester und trockener Form sehr stabil, nicht hygroskopisch, farblos und besitzen hohe Schmelz- bzw. Zersetzungspunkte. Sie lassen sich zur Anwendung als Antibiotika in Wasser, wäßrigen Puffern oder organischen Lösungsmitteln, wie z. B. Paraffinöl, lösen oder suspendieren. Besonders die Salze mit (+) Articain eignen sich für die intramuskuläre Anwendung, z.B. als Suspensionen in wäßrigen Systemen oder in physiologisch verträglichen Ölen. Galenischen Zubereitungen können die üblichen Füll- und Hilfsstoffe beigegeben werden. Die erfindungsgemäßen Produkte können auch mit anderen Wirkstoffen, z.B. dem Alkalisalz des zugrundeliegenden Cephalosporins oder anderen Cephalosporinen oder Verbindungen aus der Reihe der Chinoloncarbonsäuren, kombiniert werden. Auch die Applikationen verschiedener Diastereomeren-Salze der Cephalosporine ist möglich.

Durch die lokal anästhetische Wirkung der Articain-Komponente (B) wird bei parenteraler Gabe der erfindungsgemäßen Cephalosporinsalze Schmerz an der Applikationsstelle vermieden. Dies ist besonders bei intramuskulärer Verabreichung von großem Vorteil. Der übliche Zusatz von Lokalanästhetika ist daher entbehrlich.

Ein weiterer besonderer Vorteil der Salze besteht darin, daß sich ein Depot-Effekt erzielen läßt. Dies führt zu einer Verlängerung der Zeit, in der die Antibiotikaspiegel über der minimalen Hemmkonzentration (MHK) des Infektionserregers liegen. Für Cephalosporine ist nachgewiesen, daß die "Zeitspanne oberhalb der MHK" eine wichtige Kenngröße darstellt und am besten mit dem therapeutischen Effekt korreliert. Die absolute Höhe der Spitzenspiegel ist bei Cephalosporinen von untergeordneter Bedeutung.

Zur Bestimmung der pharmakokinetischen Eigenschaften der erfindungsgemäßen Cephalosporin-Articainsalze wurden unter anderem Untersuchungen an Mäusen und Hunden durchgeführt. Die Articain-Salze und die korrespondierenden Alkalisalze der Cephalosporine wurden in therapeutisch üblichen Dosierungen intramuskulär verabreicht. Zu festgelegten Zeitpunkten nach der Injektion des Antibiotikums wurden den Versuchstieren Blutproben entnommen und der Antibiotika-Gehalt im Blut bzw. Serum mit Hilfe des Agardiffusionstestes bestimmt. Die pharmakokinetischen Kenngrößen wurden in üblicher Weise mit Hilfe eines Computerprogramms ermittelt.

Im Vergleich zur intramuskulären Applikation einer äquivalenten Dosis des Alkalisalzes des Cephalosporins zeichnen sich die Articain-Salze z. B. von Cefotaxim, Ceftizoxim, Cefuroxim und Cefazolin durch einen deutlichen Depot-Effekt mit z. T. erheblich verlängerter Halbwertszeit aus.

Während z. B. die Halbwertszeit von Cefotaxim beim Hund 0,8 Stunden beträgt, verlängert sich die Halbwertszeit nach intramuskulärer Applikation von (+)-Articain-Cefotaxim auf 4,1 Stunden und bei dem entsprechenden (+)-Articain-Butylderivat (Beispiel 10) sogar auf 6,6 Stunden. Verlängerte Serumspiegel ermöglichen Antibiotika-Gaben in größeren zeitlichen Abständen bei gleicher therapeutischer Wirkung. Dadurch werden die Therapiekosten erheblich gesenkt und die Compliance erhöht.

Beispiel 1:

Trennung von racemischem (+)-Articain in die Enantiomeren (+) Articain und (-) Articain

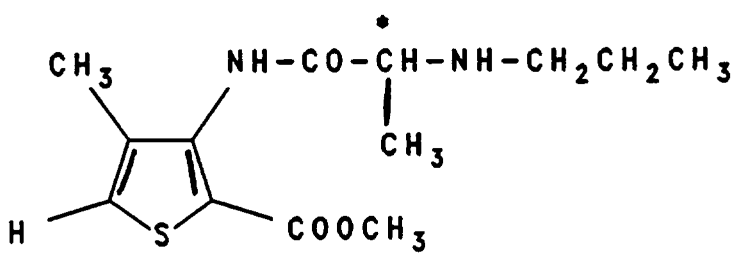

59,6 g kristalline, racemische Articainbase (Sie ist in der Literatur beschrieben, vgl. z.B.: The Merck Index, 11. ed. (1989) # 1878) (Schmp. 57° C) löst man in 200 ml siedendem Isopropanol und fügt eine siedende Lösung von 15 g L(+)-Weinsäure in 100 ml Isopropanol zu. Aus der heißen Lösung kristallisiert das (+)-Articain-Salz der L(+)-Weinsäure aus, das abgesaugt und zweimal mit Isopropanol (100 ml) ausgekocht wird (Schmp. 178-179° C).
$[\alpha]_D^{22}$ = + 14.1 (1 % in $CH_3OH$)

| $C_{13}H_{20}N_2O_3S$ x $\frac{1}{2}$ $C_4H_6O_6$ (359.4) | | | | | |
|---|---|---|---|---|---|
| ber.: | C 50.13 | H 6.45 | H 7.80 | O 26.71 | S 8.92 |
| gef.: | C 50.4 | H 6.7 | N 7.8 | O 26.6 | S 8.9 |

Dieses Salz (25,0 g) wird in 100 ml Wasser gelöst mit Sodalösung auf einen pH-Wert von ca. 9,5 gestellt und dreimal mit je 100 ml Äther extrahiert. Die vereinigten Ätherextrakte werden über wasserfreiem Natriumsulfat getrocknet und vom Lösungsmittel befreit. Man erhält in quantitativer Ausbeute (+)-Articain-Base als helles Öl, das nach längerem Stehen kristallisiert (Schmp. 37°), $[\alpha]_D^{22}$ = + 34° (1 % in $CH_3OH$) und für die Herstellung von Salzen von $\beta$-Lactam-Antibiotika direkt eingesetzt werden kann.

(-) Articain-Base wird aus der Mutterlauge isoliert. Man engt die Mutterlauge ein, nimmt den Rückstand in 200 ml Wasser auf, stellt mit Sodalösung auf pH = 9,5 und extrahiert erschöpfend mit Äther. Der mit Natriumsulfat getrocknete Extrakt wird eingeengt, dann in 200 ml Isopropanol heiß gelöst und mit einer heißen Lösung von 15 g D(-)-Weinsäure in 150 ml Isopropanol versetzt. Nach 5 Minuten saugt man den

kristallinen Niederschlag heiß ab, kocht ihn zweimal mit Isopropanol aus und trocknet im Exsiccator. Man erhält 37 g (-)-Articain-L(-)-Tartrat. Schmp. 182°C; $[\alpha]_D^{20}$ in $H_2O$ (1 %) = -14.6°. Das Freisetzen der (-)-Articain-Base geschieht nach den für (+)-Articain gemachten Angaben.

(-)-Articain

$[[\alpha]_D^{20}$ in $CH_3OH$ (1 %) = -36,9° , in Äthanol (1 %) = - 38,6°] ist ein zähflüssiges, helles Öl, das bei längerem Stehen kristallisiert (Schmp. 36-37°C) und das für die Herstellung von Salzen von Antibiotika direkt eingesetzt werden kann.

Beispiel 2

(+)-Articain-Cefotaximsalz

Eine filtrierte Lösung von 47,7 g (0,1 Mol) Cefotaxim-Natrium in 1 l Wasser (die Lösung kann auch dadurch hergestellt werden, daß man kristalline oder amorphe Cefotaximsäure mit der äquivalenten Menge Base, z.B. $NaHCO_3$, $Na_2CO_3$, NaOH, mischt) versetzt man unter Umschütteln mit einer filtrierten Lösung von 32,1 g (0,1 Mol) (±)-Articain-Hydrochlorid in 1 l Wasser. Aus der klaren Lösung beginnt nach wenigen Minuten die Abscheidung von kristallinem (+)-Articain-Cefotaximsalz. Nach einstündigem Stehen der Lösung bei Raumtemperatur kühlt man eine halbe Stunde auf ca. 5° C ab, saugt ab und wäscht den kristallinen Niederschlag mehrere Male gründlich mit Eiswasser aus, bis das Waschwasser keine Chloridionen mehr enthält. Man trocknet das Salz im Vakuum über Phosphorpentoxyd bis zur Gewichtskonstanz und erhäft 31,4 g (+) Articain-Cefotaximsalz.

Zers.-P.: 202-204 ° C $[\alpha]_D^{22}$ = + 46.8° (1 %ige Methanollösung)

| $C_{29}H_{37}N_7O_{10}S_3$ (739,9) | | | | | |
|---|---|---|---|---|---|
| ber.: | C 47,07 | H 5,05 | N 13,25 | O 21,63 | S 13,00 |
| gef.: | C 47,3 | H 5,0 | N 13,1 | O 21,6 | S 13,0 |

Engt man die Mutterlauge im Vakuum bei Raumtemperatur auf ca. 500 ml ein, so kristallisiert weiteres (+) Articain-Cefotaximsalz (ca. 4 g) aus.

Die Mutterlauge enthält (-)-Articain-Cefotaximsalz, das durch Gefriertrocknen gewonnen wird. Daraus läßt sich wie folgt (-)-Articain-Base und dessen D(-)Tartrat gewinnen: Man überschichtet die Lösung mit 300 ml Äther und setzt unter Rühren und Eiskühlung 8 g wasserfreie Soda zu, bis ein pH-Wert von ca. 9,5 erreicht ist. Man trennt den Ätherextraxt ab und wiederholt die Extraktion noch zweimal mit je 100 ml Äther. Die vereinigten Extrakte wäscht man mit 100 ml gesättigter Kochsalzlösung, trocknet über Natriumsulfat und entfernt das Lösungsmittel. Man erhält (-)-Articain-Base als helles Öl (14,0 g), aus dem durch Umsetzung mit D(-)-Weinsäure, analog den unter in Beispiel 1 genannten Bedingungen (-) Articain-D(-)-tartrat hergestellt wird:

Schmp. 182 - 184° C

| (-) Articain-D(-)-tartrat ($C_{13}H_{20}N_2O_3S$ + 1/2 $C_4H_{16}O_6$ (359.4) | | | | | |
|---|---|---|---|---|---|
| ber.: | C 50.13 | H 6.45 | N 7.80 | O 26,71 | S 8,92 |
| gef.: | C 50.6 | H 6.1 | N 7.8 | O 26.6 | S 9.1 |

Die Herstellungsbedingungen für (+) Articain-Cefotaximsalz können wegen dessen hervorgehender Stabilität, Schwerlöslichkeit und Kristallinität in einem breiten Bereich hinsichtlich Temperatur, Lösungsmittel und Konzentration variiert werden. Strebt man z.B. eine möglichst hohe Ausbeute an (+)-Articain-Cefotaximsalz an, so ist es vorteilhaft in Beispiel 2 mindestens 0,2 Mol (= 64,2 g) (± )-Articain in der angegebenen Wassermenge zuzusetzen. Die Ausbeute an (+)-Articain-Cefotaximsalz erhöht sich auf 52,0 g.

Die Kristallgröße läßt sich durch geeignete Wahl der Reaktionsbedingungen den galenischen Erfordernissen anpassen. Durch Kühlen und Rühren während des Auskristallisierens werden kleinere Kristalle erhalten. Langsameres Auskristallisieren bei Raumtemperatur oder erhöhter Temperatur liefert größere Kristalle, die durch Mahlen auf die gewünschte Korngröße gebracht werden können.

Beispiel 3

(+) Articain-Cefotaximsalz (Herstellungsvarianten)

3.1 0.477 g Cefotaxim-Natrium löst man in 10 ml Wasser und fügt eine klare wäßrige Lösung von 0.359 g (+)-Articain in Form des L(+)-weinsauren Salzes hinzu. Es scheiden sich rasch farblose Kristalle ab, die nach einer halben Stunde abgesaugt und mit Eiswasser gewaschen werden. Nach dem Trocknen über $P_2O_5$ im Vakuum erhält man 0.553 g (+)-Articain-Cefotaximsalz vom Zersetzungspunkt 202-204 °C. Die analytischen Daten entsprechen denen von Beispiel 2.

3.2 Eine filtrierte Lösung von 0.955 g Cefotaxim Natrium in 20 ml Wasser versetzt man unter Umschütteln mit einer filtrierten Lösung von 0.641 g (+) Articain-Hydrochlorid in 20 ml Wasser. Nach ca. 1 Minute beginnt die Abscheidung von kristallinem (+) Articain-Cefotaxim-Salz. Man läßt 1 Stunde bei Raumtemperatur und 0,5 Stunden im Eisbad stehen, filtriert die Kristalle ab, wäscht mit Eiswasser chloridfrei und trocknet über $P_2O_5$ im Vakuum. Ausbeute: 1.158 g; Zersetzungspunkt 202-204 °C $[\alpha]_D^{22}$ = 45.3 °C. Das Präparat ist mit Beispiel 2 identisch.

3.3 0.455 g Cefotaximsäure (hergestellt durch Ansäuern einer wäßrigen Lösung von Cefotaxim-Natrium mit 1 n HCl) suspendiert man in 5 ml Wasser und fügt eine Lösung von 0.284 g (+)-Articainbase gelöst in 5 ml Methanol zu. Man filtriert von einer kleinen Menge unlöslicher Anteile ab. Aus dem klaren Filtrat kristallisiert (+)-Articain-Cefotaximsalz. Nach 30 Minuten werden die Kristalle abfiltriert und mit einer Mischung Methanol/$H_2O$ 1:1 mehrmals gewaschen. Man erhält 0.46 g vom Zersetzungspunkt 202-204 °; Die analytischen Daten entsprechen denen von Beispiel 2.

Beispiel 4

4.1 (-) Articain-Cefotaximsalz

Eine filtrierte Lösung von 0,715 g Cefotaxim-Natrium in 15 ml Wasser versetzt man mit einer Lösung von 0,480 g (-) Articain-Hydrochlorid in 15 ml Wasser. Die Lösung wird filtriert und gefriergetrocknet. Man erhält quantitativ (-) Articain-Cefotaximsalz, das eine äquivalente Menge Kochsalz enthält. Ein kochsalzfreies Produkt wird im folgenden Beispiel 4.2 beschrieben.

4.2 1,37 g Cefotaximsäure, die man aus einer wäßrigen Cefotaxim-Natrium-Salzlösung durch Ansäuern mit 2n-Salzsäure leicht herstellen kann, suspendiert man in 100 ml Wasser und fügt langsam unter Umschütteln 0,85 g (-) Articain-Base in 20 ml Methanol zu. Anschließend entfernt man das Methanol im Vakuum und gefriertrocknet die restliche wäßrige Lösung. Man erhält quantitativ (-)-Articain-Cefotaximsalz in amorpher Form.

Beispiel 5

(+)-Articain-Ceftizoximsalz

0,864 g Ceftizoxim-Natrium (vgl. Merck Index, 11. ed. (1989) # 1949) löst man in 5 ml Wasser, filtriert und fügt eine klare Lösung von 0,64 g (+) Articain-Hydrochlorid in 5 ml Wasser zu. Nach kurzer Zeit kristallisiert das (+ )-Articain-Ceftizoximsalz aus. Man filtriert es nach einer Stunde ab, wäscht chloridfrei und trocknet die Kristalle im Vakuum über Phosphorpentoxid. Man erhält 1,02 g (+)-Articain-Ceftizoximsalz vom Zers.-P. 194-196 ° C.

| $C_{26}H_{33}N_7O_8S_3$ (667,8) | | | | |
|---|---|---|---|---|
| ber.: | C 46,76 | H 4,98 | N 14,68 | S 14,41 |
| gef.: | C 46,0 | H 4,7 | N 14,4 | S 14,1 |

Beispiel 6

(-)-Articain-Ceftizoximsalz

Die Herstellung erfolgt analog den Beispielen 4.1 und 4.2. (-)-Articain-Ceftizoximsalz ist leichter löslich in Wasser als das entsprechende Salz mit dem (+) Articain Enantiomeren.

Beispiel 7

7.1 (+) Articain-Cefuroximsalz
0,446 g Cefuroxim-Natrium (Zinacef, Merck Index, 11. ed. (1989) # 1951) löst man in 5 ml Wasser, filtriert und fügt eine klare Lösung von 0,321 g (+) Articain-Hydrochlorid in 5 ml Wasser zu. Nach kurzer Zeit kristallisiert (+ )-Articain-Cefuroximsalz aus. Nach einer Stunde filtriert man, wäscht mit Eiswasser chloridfrei und trocknet das schwerlösliche Salz im Vakuum über Phosphorpentoxid. Man erhält 0,64 g (+) Articain-Cefuroximsalz vom Zers.-P. 205-207° C.

| $C_{29}H_{36}N_6O_{11}S_2$ (708,8) | | | | |
|---|---|---|---|---|
| ber.:<br>gef.: | C 49,14<br>C 49,1 | H 5,12<br>H 4,8 | N 11,86<br>N 11,8 | S 9,05<br>S 9,3 |

7.2: 0,446 g Cefuroxim-Natrium (Zinacef, Merck Index, 11. ed. (1989) # 1951) in 5 ml Wasser versetzt man mit 5 ml einer Lösung von 0,321 g (+) Articain-Hydrochlorid. Nach wenigen Minuten kristallisiert (+) Articain-Cefuroximsalz (0,31 g) aus, das wie unter 7.1 beschrieben, isoliert wurde. Die Mutterlauge enthält das leichter lösliche (-) Articainsalz von Cefuroxim (Zers.-P. 188-190° C), das erst bei mehrstündigem Stehen auskristallisiert (vgl. Beispiel 8).

Beispiel 8

(-)-Articain-Cefuroximsalz
0,446 g Cefuroxim-Natrium (Zinacef, Merck Index, 11. ed. (1989) # 1951) löst man in 5 ml Wasser filtriert und fügt eine klare Lösung von 0,321 g (-)-Articain-Hydrochlorid in 5 ml Wasser zu. Nach ca. 20 Stunden scheidet sich (-)-Articain-Cefuroximsalzkristallin ab. Man saugt ab, wäscht mit Eiswasser chloridfrei und trocknet über Phosphorpentoxyd im Vakuum. Man erhält 0,572 g vom Zers.-P. 188-190° C.

| $C_{29}H_{36}N_6O_{11}S_2$ (708,8) | | | | |
|---|---|---|---|---|
| ber.:<br>gef.: | C 49,14<br>C 48,9 | H 5,12<br>H 4,7 | N 11,86<br>N 11,8 | S 9,05<br>S 9,1 |

Beispiel 9

9.1 (-) Articain-Cefazolinsalz
0,476 g Cefazolin-Natrium (Gramaxin, Merck Index, 11. ed. (1989), # 1925) wird in 5 ml Wasser gelöst und eine Lösung von 0,32 g (-) Articain-Hydrochlorid in 5 ml Wasser zugesetzt. Nach wenigen Minuten beginnt die Kristallisation. Nach 2 Stunden saugt man die Kristalle ab, wäscht mit Wasser chloridfrei und trocknet im Vakuum über Phosphorpentoxid. Man erhält 0,64 g (-)-Articain-Cefazolinsalz vom Zers.-P. 197-199° C.

| $C_{27}H_{34}N_{10}O_7S_4$ (738,9) | | | | |
|---|---|---|---|---|
| ber.:<br>gef.: | C 43,89<br>C 43,8 | H 4,64<br>H 4,4 | N 18,96<br>N 18,8 | S 17,36<br>S 17,2 |

9.2 (+)-Articain-Cefazolinsalz
Die Herstellung erfolgt analog Beispiel 9.1 ausgehend von 0,476 g Cefazolin-Natrium und 0,32 g (+ )-Articain-Hydrochlorid. Man erhält 0,69 g (+ )-Articain-Cefazolinsalz vom Zers.-P. 202-204° C.

| $C_{27}H_{34}N_{10}O_7S_4$ (738,9) | | | | |
|---|---|---|---|---|
| ber.: | C 43,89 | H 4,64 | N 18,96 | S 17,36 |
| gef.: | C 44,1 | H 4,3 | N 19,1 | S 17,3 |

Beispiel 10

Cefotaximsalz von (+ )n-Butyl 4-methyl-3(2-n-propylaminopropionamido) thiophen-2-carboxylat

2,385 g Cefotaxim löst man in 10 ml Wasser, setzt 10 ml Methanol zu und fügt eine klare Lösung von 1,81 g (±) n-Butyl 4-methyl-3(2-n-propylaminopropionamido) thiophene-2-carboxylat in 10 ml Wasser und 10 ml Methanol zu. Nach wenigen Minuten kristallisiert das Salz aus, das nach Kühlen des Ansatzes im Eisbad abfiltriert, viermal mit je 5 ml einer Mischung von Wasser/Methanol 1:1 chloridfrei gewaschen und im Vakuum über $P_2O_5$ getrocknet wird. Man erhält 1,75 g des im Titel genannten Salzes von Zers.-P. 198-200° C; $[\alpha]_D^{22}$ = +42,8° (1 %ige Methanollösung).

| $C_{32}H_{43}N_7O_{10}S_3$ (781.9) | | | | |
|---|---|---|---|---|
| ber.: | C 49.15 | H 5.54 | N 12.54 | S 12.3 |
| gef.: | C 49.3 | H 5,4 | N 12.4 | S 12.4 |

Die Mutterlauge enthält das leichter lösliche Cefotaximsalz mit (-)n-Butyl4-methyl-3-(2-n-propylamino-propionamido)thiophen-2-carboxylat als kationische Basenkomponente.

Beispiel 11

Cefotaximsalz von (± )-Ethyl4-methyl-3-(2-n-propylaminopropionamido)-thiophen-carboxylat

2.387 g Cefotaxim-Natrium löst man in 25 ml Wasser und fügt eine klare Lösung von 1,672 (± )-Ethyl4-methyl-3(2-n-propylaminopropionamido)thiophen-carboxylat Hydrochlorid in 25 ml Wasser zu. Die Kristallisation des im Titel genannten Salzes setzt sofort ein. Man hält eine Stunde bei Raumtemperatur und kühlt noch eine halbe Stunde im Eisbad. Die Kristalle werden dann abgesaugt, chloridfrei gewaschen mit Eiswasser und im Vakuum über $P_2O_5$ getrocknet. Man erhält 1.88 g von Zers.-P. 196-198° C.
$[\alpha]_D^{22}$ = + 44.3 (1 %ige Methanollösung)

| $C_{30}H_{39}N_7O_{10}S_3$ (753.9) | | | | | |
|---|---|---|---|---|---|
| ber.: | C 47.8 | H 5.21 | N 13.01 | O 21.22 | S 12.76 |
| gef.: | C 48.3 | H 5.2 | N 13.0 | | S 12.7 |

Das leichter lösliche Cefotaximsalz des (-) Ethyl-4-methyl-3-(2-n-propylamino-propionamido)thiophen-2-carboxylat Enantiomeren befindet sich in der Mutterlauge und wird z.B. durch Gefriertrocknung isoliert.

Beispiel 12

Cefotaximsalz von (+) n-Propyl4-methyl-3-(2-n-propylaminopropion-amido)-thiophen-2-carboxylat

2.39 g Cefotaxim-Natrium löst man in 10 ml Wasser und setzt 10 ml Methanol zu. Zu dieser Lösung gibt man eine filtrierte Lösung von 1.74 g racemischem n-Propyl-4-methyl-3-(2-n-propylaminopropionamido)-thiophen-2-carboxylat-Hydrochlorid in 10 ml Wasser und 10 ml Methanol. Es beginnt sehr rasch die kristalline Abscheidung des im Titel genannten Salzes. Man läßt den Ansatz eine Stunde bei Raumtemperatur und eine halbe Stunde unter Eiskühlung stehen, filtriert die Kristalle ab und wäscht chloridfrei durch mehrmaliges Waschen mit Wasser/Methanol 1:1. Nach dem Trocknen im Vakuum erhält man 1.4 g der Titelverbindung vom Zers.-P. 197-199° C.
$[\alpha]_D^{22}$ = + 43.8 (1 %ige Methanollösung)

| $C_{31}H_{41}N_7O_{10}S_3$ (767.93) | | | | |
|---|---|---|---|---|
| ber.: | C 48.49 | H 5.38 | N 12.77 | S 12.53 |
| gef.: | C 49.4 | H 5.1 | N 12.7 | S 12.4 |

Das Cefotaximsalz des leichter löslichen (-)n-Propyl4-methyl-3-(2-n-propylaminopropion-amido)-thiophen-2-carboxylat Enantiomeren wurde aus der Mutterlauge in bereits beschriebener Weise gewonnen.

Beispiel 13

Cefotaximsalz von (+) Benzyl4-methyl-3-(2-n-propylamino-propionamido)-thiophen-2-carboxylat

Man löst 4.77 g Cefotaxim-Natrium in 20 ml Wasser und fügt 20 ml Methanol zu. Dazu gibt man eine Lösung von 2.6 g (±)-Benzyl-4-methyl-3-(2-n-propylamino-propionamido)-thiophen-2-carboxylat in 20 ml Wasser / 20 ml Methanol und 10 ml 1n Salzsäure. Man läßt 45 Minuten bei Raumtemperatur stehen, saugt ab und wäscht 4 mal mit einer Mischung von je 10 ml Methanol/Wasser 1:1. Das abgesaugte Salz wird im Vakuum über $P_2O_5$ getrocknet. Man erhält 3.47 g der Titelverbindung vom Zers.-P. 189-191 °C.
$[\alpha]_D^{22}$ = + 39.2 (1 %ige Methanollösung)

| $C_{35}H_{41}N_7O_{10}S_3$ (816.0) | | | | | |
|---|---|---|---|---|---|
| ber.: | C 51.42 | H 5.07 | N 12.02 | O 19.61 | S 11.79 |
| gef.: | C 51.1 | H 4.9 | N 12.0 | O 19.5 | S 11.6 |

Das linksdrehende Enantiomere der verwendeten Base liefert ein leichter lösliches Cefotaximsalz, das nicht sofort auskristallisiert.,Es wird aus der Mutterlauge bei schonendem Einengen gewonnen.

Beispiel 14

Ceftriaxonsalz von n-Butyl-4-methyl-3-(2-n-propyl-aminopropionamido)-thiophen-2-carboxylat

0,657 g Ceftriaxon-Dinatriumsalz (Rocephin® Merck Index, 11. ed. 1989 # 1950) löst man in 4 ml Wasser und fügt langsam eine Lösung von 0.725 g n-Butyl4-methyl-3-(2-n-propylaminopropionamido)-thiophen-2-carboxylat-Hydrochlorid in 12 ml Wasser zu. Es scheidet sich rasch ein farbloses Salz aus, das nach NMR-Analyse auf ein Ceftriaxonmolekül zwei Moleküle der organischen Base enthält. Zers. P. ab 140 °C.

Beispiel 15

Cefotaximsalz von (±)-Methyl4,5-dimethyl-3-(2-n-propylamino-propion-amido)-thiophen-2-carboxylat-Hydrochlorid

Man löst 0.477 g Cefotaxim-Natriumsalz in 5 ml Wasser und fügt eine Lösung von 0.334 g (±)-Methyl4,5-dimethyl-3-(2-n-propylamino-propionamido)-thiophen-2-carboxylat Hydrochlorid (Lit. DE-Patentschrift 1 643 325) zu. Die Lösung wurde gefriergetrocknet. Man erhält ein farbloses Pulver der Zielverbindung, die in üblicher Weise von Kochsalz befreit wird.

Beispiel 16

16.1 Herstellung von (±) n-Butyl 4-methyl-3-(2-n-propylamino-propion-amido)-thiophen-2-carboxylat Hydrochlorid
0,73 g Natrium werden unter Rühren in 200 ml n-Butanol eingetragen und so lange zum Sieden erhitzt bis eine klare Lösung entstanden ist. Anschließend wird auf Raumtemperatur abgekühlt, 8.2 g Methyl 4-methyl-3-(2-n-propylamino-propionamido)-thiophen-2-carboxylat zugegeben und während 45 Min. entstandenes Methanol und n-Butanol abdestilliert. Die zurückbleibende gelbliche Lösung wird gekühlt und mit 1 n Salzsäure auf pH 7 gestellt, anschließend vollständig im Wasserstrahlvakuum einrotiert und zwischen Wasser und Essigester verteilt. Die Wasserphase wird noch zweimal mit Essigester extrahiert.

Die vereinigten organischen Phasen werden mit Natriumsulfat getrocknet und eingeengt. Zurück bleibt ein Öl, aus dem mit alkoholischer Salzsäure 4.5 g des Hydrochlorids vom Schmelzpunkt 173-174 °C erhalten werden.

| $C_{16}H_{26}N_2O_3S$ x HCl (392.92) | | | | |
|---|---|---|---|---|
| ber.: | C 53.0 | H 7.5 | N 7.5 | Cl 9.8 |
| gef.: | C 53.1 | H 7.5 | N 7.4 | Cl 10.1 |

In analoger Weise erhält man aus Methyl 4-methyl-3-(2-n-propylamino-propionamido)-thiophen-2-carboxylat und den entsprechenden Alkoholen:

16.2 (±) Ethyl 4-methyl-3-(2-n-propylamino-propionamido)-thiophen-2-carboxylat Hydrochlorid vom Schmelzpunkt 186-189 °C.

| $C_{14}H_{22}N_2O_3S$ x HCl (334.85) | | |
|---|---|---|
| ber.: | N 8.4 | Cl 10.6 |
| gef.: | N 8.4 | Cl 10.8 |

16.3 (±) n-Propyl 4-methyl-3-(2-n-propylamino-propionamido)-thiophen-2-carboxylat Hydrochlorid vom Schmelzpunkt 161-164 °C.

| $C_{15}H_{24}N_2O_3S$ x HCl (348.89) | | | | |
|---|---|---|---|---|
| ber.: | C 51.6 | H 7.2 | N 8.0 | Cl 10.2 |
| gef.: | C 51.9 | H 7.2 | N 8.2 | Cl 10.5 |

16.4 (±) n-Octyl 4-methyl-3-(2-n-propylamino-propionamido)-thiophen-2-carboxylat Hydrochlorid vom Schmelzpunkt 109-110 °C.

| $C_{20}H_{34}N_2O_3S$ x HCl (419.02) | | | | |
|---|---|---|---|---|
| ber.: | C 57.4 | H 8.4 | N 6.7 | Cl 8.5 |
| gef.: | C 57.8 | H 8.4 | N 6.7 | Cl 8.4 |

16.5 (±) n-Benzyl 4-methyl-3-(2-n-propylamino-propionamido)-thiophen-2-carboxylat vom Schmelzpunkt 84 °C

| $C_{19}H_{24}N_2O_3S$ (360.47) | | | |
|---|---|---|---|
| ber.: | C 63.3 | H 6.7 | N 7.8 |
| gef.: | C 63.2 | H 7.0 | N 7.7 |

Beispiel 17

(+) Articain-Hydrochlorid

2.5 g (+) Methyl-4-methyl-3-(2-n-propylamino-propionamido)-thiophen-2-carboxylat-(+)-tartrat werden in 10 ml Wasser gelöst und die Lösung mit verdünnter Natriumcarbonatlösung alkalisch gestellt. Dreimal wird dann mit Ether extrahiert. Die Etherlösung wird mit Natriumsulfat getrocknet und mit ätherischer Salzsäure das Hydrochlorid gefällt. Die überstehende Etherphase wird abdekantiert und das Hydrochlorid mit Aceton verkocht.

Ausbeute: 2.2 g vom Schmelzpunkt 169-170 °C $[\alpha]_D^{22}$ = + 16 ° (1 % in $H_2O$)

| $C_{12}H_{21}N_2O_3S$ x HCl (320.9) | | | | |
|---|---|---|---|---|
| ber.:<br>gef.: | C 48.7<br>C 49.0 | H 6.6<br>H 6.6 | N 8.7<br>N 8.4 | Cl 10.1<br>Cl 10.8 |

Beispiel 18

(-)Articain-Hydrochlorid

2.8 g (-) Methyl-4-methyl-3-(2-n-propylamino-propionamido)-thiophen-2-carboxylat-(-)-tartrat werden analog Beispiel 17 umgesetzt. Man erhält 2.5 g vom Schmelzpunkt 168-169 °C $[\alpha]_D^{22}$ = -1.6 ° (1 % in $H_2O$)

| $C_{12}H_{21}N_2O_3S$ x HCl (320.9) | | | | |
|---|---|---|---|---|
| ber.:<br>gef.: | C 48.7<br>C 49.3 | H 6.6<br>H 6.8 | N 8.7<br>N 9.7 | Cl 11.1<br>Cl 11.1 |

## Patentansprüche

**1.** Cephalosporinsalz der allgemeinen Formel (I)

$[C]^{m\ominus}$ x $[BxH^+]^{\oplus}_n$ (I)

worin

C ein salzbildendes Cephalosporin bedeutet

B ein salzbildendes Thiophenderivat der allgemeinen Formel (II) bedeutet

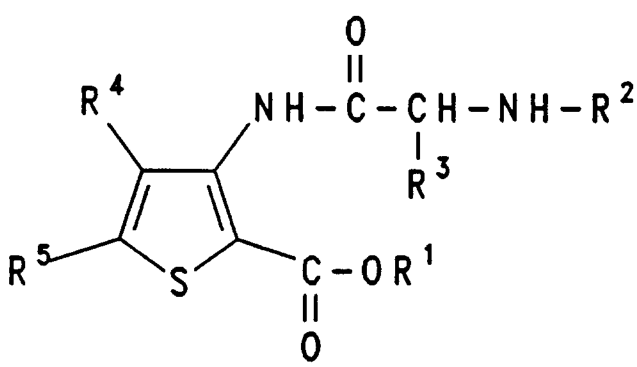

(II)

worin

$R^1$ gerade oder verzweigtes Alkyl mit 1-8 C-Atomen, das gegebenenfalls substituiert ist, vorzugsweise eine Methyl-, Äthyl-, Propyl-, Butyl- oder Benzyl-Gruppe bedeutet,

$R^2$, $R^3$, $R^4$ und $R^5$ gleich oder verschieden sein können und Wasserstoff, gerades oder verzweigtes Alkyl mit 1-4 C-Atomen, vorzugsweise Methyl bedeuten,

m eine ganze Zahl von 1 bis 3 bedeutet, und

n eine ganze Zahl von 0,1 bis 2 ist.

**2.** Cephalosporinsalz gemäß Anspruch 1, dadurch gekennzeichnet, daß C ein Cephalosporin aus der Gruppe

Cefamandol, Cefazolin, Cefoperazon, Cefbuperazon, Cefdaloxim, Cefdenir, Cefodizim, Cefixim, Cefmenoxim, Cefminox, Cefonicid, Cefotaxim, Ceforamid, Cefotetan, Cefotiam, Cefoxitin, Cefpimizol, Cefpiramid, Ceftazidim, Cefteram, Ceftezol, Ceftibuten, Ceftiofur, Ceftizoxim, Ceftriaxon, Cefuroxim,

Cefuzonam, Cephalothin, FCE 20485, Me 1206, E-0702, CP 0467, GR 69153, SR 44337, Ro 09-1428, Ro 244383, Ro 249424, Latamoxef, Flomoxef, CS 807, S 1090 bedeutet.

**3.** Cephalosporinsalz gemäß Anspruch 1, dadurch gekennzeichnet, daß C ein Cephalosporin aus der Gruppe Cefodizim, Cefotaxim, Ceftizoxim, Ceftriaxon, Cefazolin und Cefuroxim bedeutet.

**4.** Cephalosporinsalz gemäß einem der Ansprüche 1 bis 3, dadurch gekennzeichnet, daß B für das Thiophenderivat Articain steht.

**5.** Cephalosporinsalz gemäß Anspruch 4, dadurch gekennzeichnet, daß B für (+) Articain steht.

**6.** Cephalosporinsalz gemäß Anspruch 4, dadurch gekennzeichnet, daß B für (-) Articain steht.

**7.** Cephalosporinsalz gemäß Anspruch 4, dadurch gekennzeichnet, daß als Cephalosporin-Komponente C die Verbindung Cefotaxim enthalten ist.

**8.** Cephalosporinsalz gemäß Anspruch 1, dadurch gekennzeichnet, daß das Salz neben der Kationen-Komponente B mindestens eine weitere Kationen-Komponente enthält.

**9.** Cephalosporinsalz gemäß Anspruch 8, dadurch gekennzeichnet, daß neben der Kationen-Komponente B auch Alkalimetall-Kationen im Salz enthalten sind.

**10.** Verfahren zur Herstellung eines Cephalosporinsalzes der Formel (I)

$$[C]^{m\ominus} \times [BxH^{+}]^{\oplus}_{n} \qquad (I)$$

gemäß Anspruch 1, dadurch gekennzeichnet, daß man entsprechende Mengen des Cephalosporins C und der Basenkomponente (B) und gegebenenfalls weiteren Basen umsetzt.

**11.** Cephalosporinsalz gemäß Anspruch 1 zur Anwendung als Heilmittel.

**12.** Cephalosporinsalz gemäß Anspruch 1 zur Anwendung als Heilmittel mit antibiotischen Eigenschaften.

**13.** Cephalosporinsalz gemäß Anspruch 1 zur intramuskulären Anwendung.

**14.** Verwendung eines Cephalosporinsalzes gemäß Anspruch 1 zur Trennung von racemischen Basen der Struktur B gemäß Anspruch 1 in die reinen Enantiomeren.

**15.** Pharmazeutische Zubereitung enthaltend ein oder mehrere Cephalosporinsalzeder Formel (I) gemäß Anspruch 1.